# EUROPEAN PATENT APPLICATION

(11) **EP 4 079 864 A1**
(43) Date of publication of application: **26.10.2022**
(21) Application number: 21169337.9
(22) Date of filing: 20.04.2021
(51) Int. Cl.: C12Q 1/37

(54) **METHOD FOR IDENTIFYING COMPOUNDS MODIFYING THE ACTIVITY OF SARS COV2 PLPRO PROTEASE, AND USES OF THE COMPOUNDS AS IDENTIFIED**

(71) Applicant: Albert-Ludwigs-Universität Freiburg, 79085 Freiburg (DE)
(72) Inventor: Schilling, Oliver, 4052 Basel (CH); Fröhlich, Klemens, 79100 Freibug (DE); Vögele, Daniel, 77975 Ringsheim (DE); Geiss-Friedländer, Ruth, 79110 Freiburg (DE); Peters, Christoph, 79117 Freiburg (DE)
(74) Representative: Krauss, Jan

(57) **Abstract**

The present invention relates to an improved method for determining the activity of a betacoronavirus papain-like protease (PLpro) using a peptide substrate, wherein said peptide substrate comprises at least one basic amino acid in positions PI and/or PI'. The present invention furthermore relates to a method for identifying a modulator of the activity of a betacoronavirus PLpro using a substrate as above, and relates to modulators of the activity of PLpro as identifies, as well as uses thereof against betacoronaviral infection and disease.

SEQUENCE LISTING

<110>  Albert-Ludwigs-Universität Freiburg

<120>  Method for identifying compounds modifying the activity of SARS
       CoV2 PLpro protease, and uses of the compounds as identified

<130>  U31055EP

<160>  2

<170>  PatentIn version 3.5

<210>  1
<211>  5
<212>  PRT
<213>  Homo sapiens

<400>  1

Gln Arg Gln Gly Val
1               5


<210>  2
<211>  4
<212>  PRT
<213>  Homo sapiens


<220>
<221>  misc_feature
<222>  (2)..(2)
<223>  Xaa can be any naturally occurring amino acid

<400>  2

Leu Xaa Gly Gly
1

## Description

The present invention relates to an improved method for determining the activity of a betacoronavirus papain-like protease (PLpro) using a peptide substrate, wherein said peptide substrate comprises at least one basic amino acid in positions PI and/or P1'. The present invention furthermore relates to a method for identifying a modulator of the activity of a betacoronavirus PLpro using a substrate as above, in particular a peptide derived from SUMO1, and relates to modulators of the activity of PLpro as identified, in particular with SUMO1 as a substrate, as well as uses thereof against betacoronaviral infection and disease.

### Background of the invention

Betacoronaviruses (β-CoVs or Beta-CoVs) are one of five genera of coronaviruses of the subfamily *Orthocoronavirinae* in the family *Coronaviridae,* of the order *Nidovirales.* They are enveloped, positive-sense, single-stranded RNA viruses of zoonotic origin.

After human coronavirus 229E (HCoV-229E) (classified in the genus *Alphacoronavirus*) and HCoV-OC43 (*Betacoronavirus* lineage 2a member) was described in the 1960s, SARS-CoV-1 (*Betacoronavirus* lineage 2b member) that emerged in March 2003, HCoV-NL63 (*Alphacoronavirus* lineage 1b member) described in 2004, HCoV-HKU1 (*Betacoronavirus* lineage 2a member) discovered in 2005, and finally MERS-CoV that emerged in 2012 (classified in *Betacoronavirus* lineage 2c), the coronavirus that emerged in the Chinese city of Wuhan in December 2019 is the seventh human coronavirus described to date as being responsible for respiratory infection.

Evidence was rapidly reported that patients were suffering from an infection with a novel *Betacoronavirus* tentatively named 2019 novel coronavirus (2019-nCoV). Despite drastic containment measures, the spread of 2019-nCoV, now officially known as severe acute respiratory syndrome coronavirus 2 (SARS-CoV-2), is ongoing. Phylogenetic analysis of this virus indicated that it is different (∼80% nucleotide identity) but related to SARS-CoV-1.

Coronaviruses feature the largest known RNA virus genomes ranging approximately from 26 to 32 kb, containing at least 6 (14 in case of SARS-CoV-2) open reading frames (ORFs). The major reading frame ORF 1ab encodes for two overlapping polyproteins (ppla, pplab), which are cleaved into 16 non-structural proteins (nsp1-16) by the main protease Mpro (also referred to as 3CLpro) and the papain-like protease PLpro. In addition, the papain-like protease is also a deubiquitinase. The remainder of the genome encodes for accessory and structural proteins such as the spike glycoprotein (S), envelope protein (E), membrane protein (M) and the nucleocapsid phosphoprotein (N).

The SARS-CoV-2 main protease is considered a promising drug target, as it is dissimilar to human proteases. Sequence and structure of the main protease are closely related to those from other betacoronaviruses, facilitating drug discovery attempts based on previous lead-compounds.

Ullrich S, and Nitsche C. (in: The SARS-CoV-2 main protease as drug target. Bioorg Med Chem Lett. 2020 Sep 1;30(17):127377. doi: 10.1016/j.bmcl.2020.127377. Epub 2020 Jul 2. PMID: 32738988; PMCID: PMC7331567) investigate covalently binding peptidomimetics and small molecules. Various compounds show antiviral activity in infected human cells. Potential problems associated with limited drug-likeness of peptidomimetics could be circumvented by pursuing alternative small molecules, which might, for example, be accessible from fragment-based drug discovery campaigns. Small molecules like ebselen or carmofur are Mpro inhibitors with anti-SARS-CoV-2 activity in cells; however, their thiol reactivity might prove challenging. Repurposing approved protease inhibitors is an alternative approach, but attempts to repurpose the approved combination of HIV protease inhibitors, ritonavir and lopinavir, was unsuccessful in clinical studies.

Coronavirus PLpro has a dual pathophysiological function: The protease is required for maturation of the viral polypeptide precursor and it antagonizes innate cellular antiviral responses. The latter function stems from the enzyme's ability to deubiquitinate and de-ISG15ylate proteins in antiviral signaling pathways [2].

SUMO proteins are small polypeptides of around 12 kDa that can be covalently conjugated to specific lysine residues on target proteins through a process called SUMOylation. SUMO proteins are highly conserved across all eukaryotes, and to date, five genes encoding potential SUMO paralogs have been identified in humans. SUMO1, 2 and 3 are ubiquitously expressed, SUMO1 shares only 47% sequence identity with SUMO2/3 [Lork, M.; Lieber, G.; Hale, B.G. Proteomic Approaches to Dissect Host SUMOylation during Innate Antiviral Immune Responses. Viruses 2021, 13, 528. https://doi.org/10.3390/v13030528]. In contrast, human SUMO1 shares 100% aa sequence identity with the mouse ortholog.

Kouznetsova VL, et al. (in: Potential COVID-19 papain-like protease PLpro inhibitors: repurposing FDA-approved drugs. PeerJ. 2020 Sep 18;8:e9965. doi: 10.7717/peerj.9965. PMID: 32999768; PMCID: PMC7505060) describe that, using the crystal structure of SARS-CoV-2 papain-like protease PLpro as a template, they developed a pharmacophore model of functional centers of the PLpro inhibitor-binding pocket. With this model, they conducted data mining of the conformational database of FDA-approved drugs. This search identified 147 compounds that can be potential inhibitors of SARS-CoV-2 PLpro. The conformations of these compounds underwent 3D fingerprint similarity clusterization, followed by docking of possible conformers to the binding pocket of PLpro. Docking of random compounds to the binding pocket of protease was also done for comparison. Free energies of the docking interaction for the selected compounds were lower than for random compounds. The drug list obtained includes inhibitors of HIV, hepatitis C, and cytomegalovirus (CMV), as well as a set of drugs that have demonstrated some activity in MERS, SARS-CoV, and SARS-CoV-2 therapy. They report on two other studies where docking experiments were used to predict binding of existing pharmaceuticals to the SARS-CoV-2 PLpro (Devaraj SG, Wang N, Chen Z, Chen Z, Tseng M, Barretto N, Lin R, Peters CJ, Tseng CT, Baker SC, Li K. Regulation of IRF-3-dependent innate immunity by the papain-like protease domain of the severe acute respiratory syndrome coronavirus. J Biol Chem. 2007 Nov 2;282(44):32208-21. doi: 10.1074/jbc.M704870200. Epub 2007 Aug 30. PMID: 17761676; PMCID: PMC2756044; World Health Organization (2020) World Health Organization Coronavirus disease (COVID-19): situation report-171. 2020. https://www.who.int/docs/default-source/coronaviruse/situation-reports/20200709-covid-19-sitrep-171.pdf?sfvrsn=9aba7ec7_2https://www.who.int/docs/default-source/coronaviruse/situation-reports/20200709-covid-19-sitrep-171.pdf?sfvrsn=9aba7ec7_2). They recommend testing of the selected compounds for treatment of COVID-19.

It is an object of the present invention to provide novel approaches in order to identify and thus provide specific and compounds interfering with the pathophysiological functions of a betacoronavirus papain-like protease PLpro, in particular the PLpro of SARS CoV2. Other objects and advantages will become apparent to the person of skill when studying the present description of the present invention.

In a first aspect of the present invention, the above object is solved by a method for determining the activity of a betacoronavirus papain-like protease (PLpro), comprising the steps of contacting a peptide substrate for said PLpro with said PLpro, measuring cleaving of said peptide substrate by said PLpro, and determining the activity of said PLpro based on said cleaving, wherein said peptide substrate comprises at least one basic amino acid in positions P1 and/or P1' according to the Schechter and Berger nomenclature.

In a second aspect of the present invention, the above object is solved by a method for identifying a modulator of the activity of a betacoronavirus papain-like protease PLpro, comprising the steps of contacting a peptide substrate for said PLpro with said PLpro in the presence of at least one candidate modulator, detecting cleaving of said peptide substrate by said PLpro, comparing cleaving of said peptide substrate in the absence of the at least one candidate modulator, and identifying a modulator of the activity of said PLpro based on said comparing, wherein said peptide substrate comprises at least one basic amino acid in positions P1 and/or P1' according to the Schechter and Berger nomenclature. In one aspect, the peptide substrate comprises human SUMO1 or a fragment thereof comprising the amino acid sequence QRQGV (SEQ ID NO: 1) of SUMO1.

In a third aspect of the present invention, the above object is solved by a method for identifying at least one compound as suitable for the treatment of a betacoronaviral infection, comprising performing the method according to the present invention, and detecting a reduction of betacoronaviral infection in the presence of said modulator compared to infection in the absence of said modulator. In one aspect, the peptide substrate comprises human SUMO1 or a fragment thereof comprising the amino acid sequence QRQGV (SEQ ID NO: 1) of SUMO1.

In a fourth aspect of the present invention, the above object is solved by a method for producing a pharmaceutical composition effective against betacoronaviral infection, comprising performing a method according to the present invention, and suitably formulating said compound as identified into a pharmaceutical composition.

In a fifth aspect of the present invention, the above object is solved by a method for treating a betacoronaviral infection in a subject in need thereof, comprising administering a pharmaceutical composition as produced according to the present invention to said subject. This includes the use of an inhibitor of the activity of a betacoronavirus papain-like protease PLpro on SUMO1 in the treatment or prevention of a betacoronaviral infection in a subject.

In a sixth aspect of the present invention, the above object is solved by a kit comprising materials for performing a method according to the present invention in one or separate containers, optionally together with auxiliary agents and/or instructions for performing said method.

In a sixth aspect of the present invention, the above object is solved by the use of the kit according to the present invention for a method according to the present invention.

It was surprisingly found that SARS CoV2 PLpro exhibits a yet unknown preference for basic amino acids in the positions P1 and/or P1' as well as in the vicinity (i.e. between P4 and P4') of the cleavage site of a peptide. This provides for improved activity assays of betacorona-PLpro and allows the design of improved assays for identifying antiviral SARS CoV2 PLpro inhibitors.

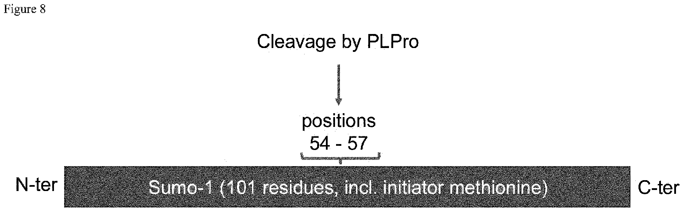

It was furthermore surprisingly found that SARS CoV2 PLpro exhibits a yet unknown preference for binding and cleaving mammalian SUMO1, in particular human SUMO1, and a processing of SUMO at about position 54/55 thereof (Figure 8). Without wanting to be bound by theory, the interaction appears to be in agreement with the above principles. This provides for the design of assays for identifying specific antiviral SARS CoV2 PLpro inhibitors targeting the interaction with SUMO1.

Lork, Lieber, and Hale (in: Proteomic Approaches to Dissect Host SUMOylation during Innate Antiviral Immune Responses. Viruses 2021, 13, 528. https://doi.org/10.3390/v13030528) disclose infection with influenza A virus (IAV) results in a global increase in cellular SUMOylation in various cell lines, while no substantial differences in the abundance of proteins of the SUMOylation machinery (UBC9, SAE1/2) were detected. Proteomic analyses also uncovered that there was a widespread loss of SUMO from many substrates during infection, which was surprising given that the overall levels of SUMOylation appeared to increase during IAV infection when assayed by western blot. This discrepancy may be explained by the SUMOylation of viral proteins, such as NS1, M1 and NEP, that were identified as SUMO targets and which are abundantly expressed during infection. Additional SUMO proteomic studies on cells infected with influenza B virus, or a genetically-modified mutant IAV lacking its major IFN-antagonist protein, revealed that the widespread loss of SUMOylated proteins during IAV infection is generally conserved during these other virus infections. They disclose that it further remains unclear how global changes in SUMOylation are triggered in the context of innate immune responses (e.g., cellular stress or direct induction by viral proteins). Finally, they speculate that delineating the molecular mechanisms of SUMO pathways might open up novel opportunities for therapeutic interventions. For example, viruses that depend on SUMO-related mechanisms for replication could be inhibited by specific targeting of required SUMO-related host proteins. The observation that absence of SUMO2/3 results in a spontaneous IFN signature also suggests that the SUMOylation machinery might be a relevant target for autoimmune or auto-inflammatory therapeutics.

Coronavirus, and in particular SARS CoV2 encodes for the protease PLpro. PLpro is a cysteine protease, and its activity consists in (i) the recognition of the LXGG (SEQ ID NO: 2) motif and the subsequent hydrolysis of the peptide bond on the carboxyl side of glycine in the PI position that results in the release of the NSP1, NSP2, and NSP3 proteins; (ii) deubiquitination; and (iii) deISGylation, i.e., the removal of the ubiquitin-like protein interferon-induced gene 15 from host proteins. It is noteworthy that these latter two activities interfere with the innate immune response to viral infection (Rut W, Lv Z, Zmudzinski M, Patchett S, Nayak D, Snipas SJ, E1 Oualid F, Huang TT, Bekes M, Drag M, Olsen SK. Activity profiling and crystal structures of inhibitor-bound SARS-CoV-2 papain-like protease: A framework for anti-COVID-19 drug design. Sci Adv. 2020 Oct 16;6(42):eabd4596).

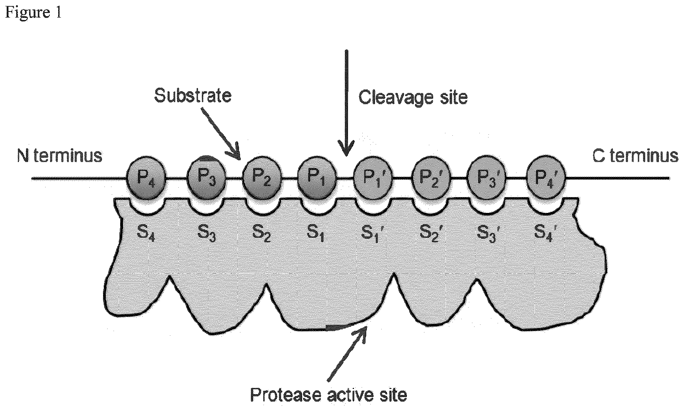

The basis for the specificity of a protease are interactions between enzyme and substrate, often at several amino acid positions close to the active site of the enzyme. This is described by the nomenclature of Schechter and Berger (e.g. Figure 1). Despite the number of attempts to study the function and structure of SARS CoV2 PLpro, no preference for basic amino acid residues in PI and/or P1' has been described (6-13).

Similarly, biochemical binding studies have not revealed such a preference of PLpro, and in particular SARS CoV2 PLpro for basic residues in P1 and/or P1' (6, 7). The preference for the deISGylation activity for SARS CoV2 PLpro was connected to a preference for di-glyine in P2 and P12.

In a first aspect of the present invention, the above object is solved by a method for determining the activity of a betacoronavirus papain-like protease PLpro, comprising the steps of contacting a peptide substrate for said PLpro with said PLpro, measuring cleaving of said peptide substrate by said PLpro, and determining the activity of said PLpro based on said cleaving, wherein said peptide substrate comprises at least one basic amino acid in positions P1 and/or P1' according to the Schechter and Berger nomenclature. This method provides for improved activity assays of betacorona-PLpro.

In a second aspect of the present invention, the above object is solved by a method for identifying a modulator of the activity of a betacoronavirus papain-like protease PLpro, comprising the steps of contacting a peptide substrate for said PLpro with said PLpro in the presence of at least one candidate modulator, detecting cleaving of said peptide substrate by said PLpro, comparing cleaving of said peptide substrate in the absence of the at least one candidate modulator, and identifying a modulator of the activity of said PLpro based on said comparing, wherein said peptide substrate comprises at least one basic amino acid in positions P1 and/or P1' according to the Schechter and Berger nomenclature. In one aspect, the peptide substrate comprises human SUMO1 or a fragment thereof comprising the amino acid sequence QRQGV (SEQ ID NO: 1) of SUMO1. This assay allows the design of improved assays for identifying PLpro modulators, and in particular antiviral SARS CoV2 PLpro inhibitors. Preferred is therefore the method according to the present invention, wherein said modulator is an inhibitor of the activity of a betacoronavirus papain-like protease PLpro.

A preferred embodiment of the invention relates to method for identifying a modulator of the activity of a betacoronavirus papain-like protease PLpro with SUMO1 protein, comprising the steps of contacting a peptide substrate comprising SUMO1 protein or a fragment thereof that can be cleaved by PLpro, preferably including the the amino acid sequence QRQGV (SEQ ID NO: 1) of SUMO1, with said PLpro in the presence of at least one candidate modulator, detecting cleaving of said peptide substrate by said PLpro, comparing cleaving of said peptide substrate in the absence of the at least one candidate modulator, and identifying a modulator of the activity of said PLpro based on said comparing, This assay allows the identification of PLpro/SUMO1 modulators, and in particular antiviral SARS CoV2 PLpro inhibitors of processing of SUMO1. Preferred is therefore the method according to the present invention, wherein said modulator is an inhibitor of the activity of a betacoronavirus papain-like protease PLpro. It is expected that these modulators and/or inhibitors will have antiviral activities (see above).

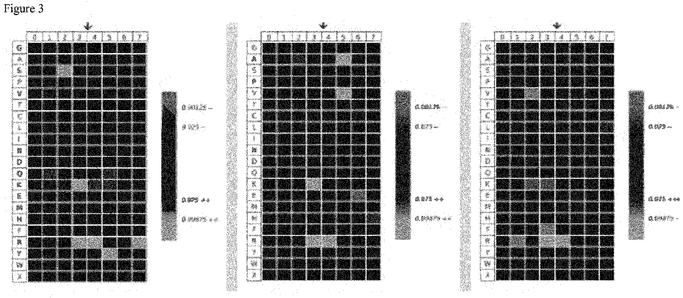

As mentioned above, the basis for the specificity of a protease are interactions between enzyme and substrate, often at several amino acid positions close to the active site of the enzyme (e.g. in particular at between S4 to S4', see Figure 1). Preferred is therefore a method according to the present invention, wherein said peptide substrate comprises at least one additional basic amino acids in positions P2 to P3 and P2' to P4' thereof, and preferably one or two additional basic amino acids in positions P3' and/or P4'. Preferred examples, not only for basic amino acids, can be found in Figures 3 to 7, and Table 1. Particularly preferred are amino acids K, R, and G for P1, and K, R, S, and A for P1' (single letter code). Further preferred are R, V, or C for P3, A, V, F, Y, S, or N for P2, R, V, G, A. Y or C for P2', K or R for P3', and R, H or P for P4'. Also, modified amino acids can be used as long as they have basic characteristics.

Preferred is a method according to the present invention, wherein said basic amino acids are selected from lysine and/or arginine, and/or wherein said peptide substrate comprises a basic amino acids in both positions P1 and P1' thereof, preferably lysine/lysine (K/K) or arginine/arginine (R/R). Another possibility is a mix of K/R, or R/K

The peptide substrate for the method according to the present invention needs to be long enough to be effectively cleaved by the PLpro enzyme, preferably under the conditions (pH 6.0 or 8.0) as described herein. Preferred is a substrate having a length as described for the peptide library as described herein, more preferably a length of between 4 to 20 amino acids, preferably of between 4 and 10 amino acids. Furthermore, it is advantageous that the P1 and P1' are located approximately in the middle of said peptide part of the substrate.

Apart from the conditions as mentioned herein, the peptide substrate can have any suitable amino acid sequence that still allows for an effective assay (i.e. solubility and pH compatibility as well as attachment of labels, markers and/or other groups as described below), i.e. the peptide can have an amino acid sequence of a naturally occurring protein (such as human, mouse, or viral), and preferably said peptide substrate comprises human SUMO1 or a fragment thereof comprising the amino acid sequence QRQGV (SEQ ID NO: 1) of SUMO1. Alternatively, the peptide is synthetically/chemically produced with a specific or arbitrary sequence (again apart from the requirements as described herein). Methods for peptide synthesis are well known to the person of skill. Again, modified amino acids may be used.

In the methods of the present invention, cleavage of the peptide substrate (such as SUMO1 or a fragment thereof comprising the amino acid sequence QRQGV (SEQ ID NO: 1) of SUMO1) as provided is detected, either in order to determine the activity of the enzyme, or a mutant or modified derivative enzyme thereof, or to identify a modulator, preferably inhibitor, of the protease activity, i.e. the cleavage of the peptide substrate. In general, therefore, the cleaved (or-non cleaved) peptide substrate or the parts thereof need to be detected, either qualitatively (as mostly required for the screening) and/or quantitatively (as required for activity assays). While the peptide fragments could be detected themselves, e.g. because of their mass or charge or size, preferred is a method according to the present invention, wherein said peptide substrate comprises a label suitable for detecting a cleaving of said substrate. Any suitable label not interfering with cleavage can be used, e.g. attached directly or through a linker molecule to said substrate, such as, for example, a fluorophore and quencher, a FRET biosensor, mass labels for mass-spectrometry, enzymes, antigens, metal particles, and dyes. Respective labels and markers for peptide analysis are well known to the person of skill.

Further preferred is the method according to present invention, wherein the betacoronavirus PLpro is from HCoV-OC43, SARS-CoV-1, HCoV-HKU1, MERS-CoV or SARS-CoV-2. Also included are PLpro peptidase enzymes the amino acid sequence of which is identical to at least 80%, preferably to at least 90%, and more preferably to at least 95% identical to PLpro from HCoV-OC43, SARS-CoV-1, HCoV-HKU1, MERS-CoV or SARS-CoV-2, and further has a substantial peptidase activity.

Preferred is the method according to the present invention, wherein said method is performed or conducted in a high-throughput format, e.g. using multiple well plates, compound libraries, automation, and/or robotics. Respective formats are known to the person of skill and can be readily adjusted to the present requirements/circumstances.

As described herein, the method according to the present invention identifies a modulator of the activity of PLpro, in the present context the proteinase activity cleaving the peptide substrate. This activity is modulated, if a difference is identified between an assay where activity is determined in the presence of the (potential) at least one modulator compound and a substantially identical assay, but without the at least one modulator compound (control). Modulators can increase (useful for the activity assay format) or decrease the activity (inhibitor). The activity can be modulated to at least 5%, at least 10%, at least 20%, at least 30%, at least 50%, at least 75% or more, either increased or decreased, up to a complete loss of activity in case of an inhibitor. For activators, there is no clear limit, except for the thermodynamics of the reaction. For inhibitors, at least more than 50% is preferred, as this will lead to a loss of the virus over time.

For identifying the modulator, any suitable compound can be tested, preferred is the method according to the present invention, wherein said modulator is at least one compound selected from a proteinaceous domain, a small molecule, a peptide, a peptide library, antibodies or antigen binding fragments thereof, an environmental substance, probiotic, toxin, aerosol, medicine, nutrient, galenic composition, plant extract, volatile compound, homeopathic substance, incense, pharmaceutical drug, drug to be repurposed, vaccine, a compound or compound mixture derived from organisms, for example animals, plants, fungi, bacteria, archaea, a chemical compound, a compound used in food or cosmetic industry, and a library of said compounds, and preferably a small molecule (i.e. smaller than about 500 Dalton).

Yet another important aspect then related to an inhibitor of the activity of a betacoronavirus papain-like protease PLpro, comprising a peptide part mimicking a PLpro peptide substrate, comprising at least one basic amino acid in positions PI and/or P1' according to the Schechter and Berger nomenclature, wherein said inhibitor substantially covalently or covalently binds to said PLpro and or the vicinity thereof, thereby inhibiting the protease activity. While the general structure of the amino acid sequence of the peptide part of the inhibitor is not substantially different from the above described substrate, the inhibitor will effectively bind to the PLpro enzyme based on the improved characteristics based on the basic amino acid(s), and hence mimic the substrate, the inhibitor in addition comprises modifications that lead to a covalent binding to said PLpro in a way to permanently block the active site. Covalent inhibitors are known (Delre P, Caporuscio F, Saviano M, Mangiatordi GF. Repurposing Known Drugs as Covalent and Non-covalent Inhibitors of the SARS-CoV-2 Papain-Like Protease. Front Chem. 2020;8:594009. Published 2020 Nov 16. doi:10.3389/fchem.2020.594009), but do not comprise basic amino acids. Covalent binding may occur through the side groups of amino acids involved (e.g. cysteine) and/or active chemical groups or entities that are attached to the peptide, e.g. instead of the label as used for the assay substrates, which is preferably not used in this embodiment. The substrate thus may be used as a targeted drug fused or conjugated to the actual peptidase inhibitor.

One preferred aspect is an inhibitor of the activity of a betacoronavirus papain-like protease PLpro on SUMO1, and/or the use thereof in the treatment or prevention of a betacoronaviral infection in a subject.

Preferred is the inhibitor according to the present invention, wherein said peptide substrate comprises at least one additional basic amino acids in positions P2 to P3 and P2' to P4' thereof, and preferably one or two additional basic amino acids in positions P3' and/or P4'.

Further preferred is the inhibitor according to the present invention, wherein said basic amino acids are selected from lysine and/or arginine, and/or wherein said peptide substrate comprises a basic amino acids in both positions P1 and P1' thereof, preferably lysine/lysine or arginine/arginine. In the inhibitor, the peptide substrate can be as above, e.g. has a length of between 4 to 20 amino acids, preferably of between 4 and 10 amino acids, and wherein preferably P1 and P1' are located in the middle of said substrate. In the inhibitor the peptide substrate can have an amino acid sequence of a naturally occurring protein, such as comprising human SUMO1 or a fragment thereof comprising the amino acid sequence QRQGV (SEQ ID NO: 1) of SUMO1, or wherein said peptide is synthetically produced.

Yet another aspect of the present invention then relates to a method for identifying at least one compound as suitable for the treatment of a betacoronaviral infection, comprising performing the method according to the present invention as disclosed above, and detecting a reduction of betacoronaviral infection in the presence of said modulator compared to infection in the absence of said modulator, wherein said reduction indicates a compound as suitable. Preferably, the betacoronaviral infection is with HCoV-OC43, SARS-CoV-1, HCoV-HKU1, MERS-CoV or SARS-CoV-2. Further preferred is a method, wherein the peptide substrate is human SUMO1 or a fragment thereof comprising the amino acid sequence QRQGV (SEQ ID NO: 1) of SUMO1.

Respective assays to test for betacoronaviral infection are known in the art, and can include in vivo and/or in vitro models, most assays test for a change in viral load, e.g. using suitable PCR methods or antibody-based reactions.

Yet another aspect of the present invention then relates to a method for producing a pharmaceutical composition effective against betacoronaviral infection, comprising performing a method according to the present invention or providing the inhibitor according to the present invention as above, and suitably formulating said compound as identified into a pharmaceutical composition. Preferably, said compound is a modulator, such as an inhibitor, more preferably a specific and/or selective inhibitor of processing of SUMO1 by PLpro.

Therefore, in this aspect of the present invention, the modulator, and in particular the inhibitor can be formulated into/provided and/or is administered as a suitable pharmaceutical composition, such as a tablet, capsule, granule, powder, sachet, reconstitutable powder, dry powder inhaler and/or chewable. Such solid formulations may comprise excipients and other ingredients in suitable amounts. Such solid formulations may contain e.g. cellulose, cellulose microcrystalline, polyvidone, in particular FB polyvidone, magnesium stearate and the like.

It is to be understood that the present modulator and/or a pharmaceutical composition comprising the present modulator is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the modulator, if required, as long as they act in combination (i.e. directly and/or indirectly, preferably synergistically) with the present modulator.

Thus, the modulator of the invention can be used alone or in combination with other active compounds - for example with medicaments already known for the treatment of the coronaviral diseases, whereby in the latter case a favorable additive, amplifying or preferably synergistically effect is noticed. Suitable amounts to be administered to humans range from 5 to 500 mg, in particular 10 mg to 100 mg.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for modulators, and for example peptide inhibitors, and will readily be able to choose suitable pharmaceutically acceptable carriers or excipients, depending, e.g., on the formulation and administration route of the pharmaceutical composition.

The therapeutics can be administered orally, e.g. in the form of pills, tablets, coated tablets, sugar coated tablets, hard and soft gelatin capsules, solutions, syrups, emulsions or suspensions or as aerosol mixtures. Administration, however, can also be carried out rectally, e.g. in the form of suppositories, or parenterally, e.g. in the form of injections or infusions, or percutaneously, e.g. in the form of ointments, creams or tinctures.

In addition to the aforementioned modulator and in particular inhibitor of the invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more modulator of the invention and also other therapeutically active substances as described herein.

Preferred is the modulator according to the present invention, wherein said modulator is administered to said subject in an effective dosage, for example of 45 mg daily, taken as 22.5 mg BID in the morning 15-60 min before a meal, and in the evening at least 2 hours after any meal. Nevertheless, this exemplary dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight particularly 1 to 50 mg/kg. Suitable dosage rates for larger mammals, for example humans, are of the order of from about 10 mg to 3 g/day, conveniently administered once or in divided doses, e.g. 2 to 4 times a day, or in sustained release form. In general, a daily dose of approximately 10 mg to 100 mg, particularly 10 to 50 mg, per human individual is appropriate in the case of the oral administration. In the case of other administration forms too, the daily dose is found within similar ranges.

Yet another aspect of the present invention then relates to a method for treating or preventing a disease based on betacoronaviral infection in a subject in need thereof, such as COVID-19, comprising performing the method according to the present invention as above or providing a modulator, preferably an inhibitor as above or a pharmaceutical composition as produced as disclosed above, and administering an effective amount of the modulator, preferably the inhibitor, or the pharmaceutical composition to said subject.

Yet another aspect of the present invention then relates to a method for treating or preventing a betacoronaviral infection in a subject in need thereof, comprising performing the method according to the present invention as above or providing a modulator, preferably an inhibitor as above or a pharmaceutical composition as produced as disclosed above, and administering an effective amount of the modulator, preferably the inhibitor, or the pharmaceutical composition to said subject.

Preferred is the method according to the present invention, wherein said viral infection is betacoronaviral infection and is selected from an infection with HCoV-OC43, SARS-CoV-1, HCoV-HKU1, MERS-CoV or SARS-CoV-2, with the latter being preferred.

Preferred is the method according to the present invention, wherein said disease and/or viral infection involves and is caused by, to at least a substantial amount, the activity of a betacoronavirus papain-like protease PLpro on SUMO1, for example cleaving of SUMO 1 by PLpro.

By "treatment" or "treating" is meant any treatment of a disease or disorder, in a subject, such as a mammal or human, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

Treatment can include co-treatment according to standard antiviral therapy (SAT), for example selected from at least one of neuraminidase inhibitors (e.g., oseltamivir, zanamivir), favipiravir, remdesivir, ribavirin (tribavirin), interferon alfa-2b/ribavirin systemic, interferon alpha 2a or 2b, inclusive any pegylated versions, chloroquine or hydroxychloroquine (given in combination with azithromycin), dolutegravir + rilpivirine (JULUCA^{®}), dolutegravir + lamivudine (DOVATO^{®}), ritonavir + lopinavir (Kaletra^{®}), bictegravir + tenofovir alafenamide + emtricitabine (BIKTARVY^{®}), dolutegravir + abacavir + lamivudine (TRIUMEQ^{®}), elvitegravir + cobicistat +emtricitabine + tenofovir alafenamide (GENVOYA^{®}), and elvitegravir + cobicistat + emtricitabine + tenofovir disoproxil fumarate + emtricitabine (STRIBILD^{®}). Preferred is remdesivir, chloroquine and/or hydroxychloroquine or a combination thereof.

Yet another aspect of the present invention then relates to a kit comprising materials for performing a method according to the present invention in one or separate containers, optionally together with auxiliary agents and/or instructions for performing said method. The kit may comprise one or more peptide substrates as above, PLpro enzyme, relevant antibodies, recombinant marker proteins, respective peptides, dyes and other labels, as well buffers and matrices for performing the tests.

Preferred is the use of the kit according to the present invention for a method according to the present invention, i.e. for determining the activity of a betacoronavirus papain-like protease PLpro, for identifying a modulator of the activity of a betacoronavirus papain-like protease PLpro, and/or for identifying at least one compound as suitable for the treatment of a betacoronaviral infection, as described herein.

Yet another aspect of the present invention then relates to an inhibitor of the activity of a betacoronavirus papain-like protease PLpro on SUMO1 for use in the treatment or prevention of a betacoronaviral infection in a subject in need thereof, as disclosed herein.

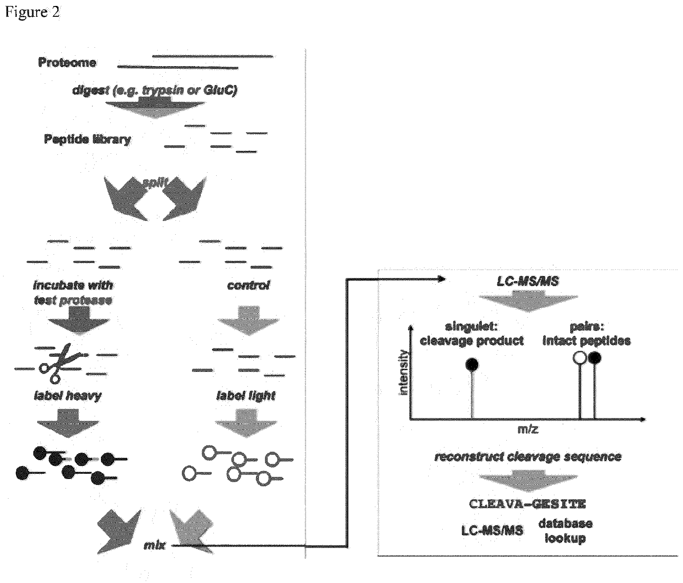

As a proof of concept, the inventors have recombinantly produced SARS CoV2 PLpro protease, and commercially obtained the protease as an external control, respectively (Boston Biochem). An unbiased specificity screening for endoproteolytic activity of SARS CoV2 PLpro was performed, as shown in Figure 2. Respective peptide libraries were produced with GluC or chymotrypsin, in order to also be able to identify an eventual preference for basic amino acids.

Using the recombinant protease as produced, at pH 6.0 and 8.0 a clear preference for basic amino acids (Arg or Lys) in P1 or P1' (Figure 3, Figure 4) was found, which was confirmed using the external control SARS CoV2 PLpro (Boston Biochem) (Figure 5, Figure 6).

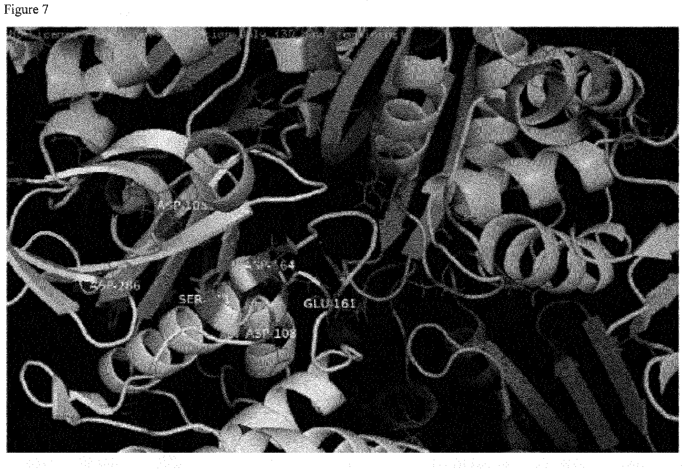

A subsequent structural analysis of PLpro identified numerous acidic amino acids in the vicinity of the active center (in particular at between S4 to S4', see Figure 1) which can contribute to binding of basic amino acids (see Figure 7).

In another example, it was tested whether SARS CoV2 PLpro can also cleave proteins instead of only peptides at the basic residues in P1 or P1'. A lung cell pellet was lysed using the detergent RapiGest; another lysate was mechanically lysed only in 10 mM HEPES buffer. Both lysates were digested with PLpro and subsequently with GluC. Semi-specific peptides with > 50 % enrichment upon SARS CoV2 PLpro incubation were taken into account. In P1 and P1', basic amino acids were identified (Figure 8). Of interest is furthermore the processing of SUMO at and around position 54.

In the context of the present invention, "about" shall mean +/- 10% of the value as given, unless indicated otherwise.

The present invention in particular relates to the following items.
Item 1. A method for determining the activity of a betacoronavirus papain-like protease (PLpro), comprising the steps of contacting a peptide substrate for said PLpro with said PLpro, measuring cleaving of said peptide substrate by said PLpro, and determining the activity of said PLpro based on said cleaving, wherein said peptide substrate comprises at least one basic amino acid in positions P1 and/or P1' according to the Schechter and Berger nomenclature.
Item 2. The method according to Item 1, wherein said peptide substrate comprises at least one additional basic amino acids in positions P2 to P3 and P2' to P4' thereof, and preferably one or two additional basic amino acids in positions P3' and/or P4'.
Item 3. The method according to Item 1 or 2, wherein said basic amino acids are selected from lysine and/or arginine, and/or wherein said peptide substrate comprises a basic amino acids in both positions P1 and P1' thereof, preferably lysine/lysine or arginine/arginine.
Item 4. The method according to any one of Items 1 to 3, wherein said peptide substrate has a length of between 4 to 20 amino acids, preferably of between 4 and 10 amino acids, and wherein preferably P1 and P1' are located in the middle of said substrate.
Item 5. The method according to any one of Items 1 to 4, wherein said peptide substrate has an amino acid sequence of a naturally occurring protein, or wherein said peptide is synthetically produced.
Item 6. The method according to any one of Items 1 to 5, wherein said peptide substrate comprises a label suitable for detecting a cleaving thereof, such as, for example, a fluorophore and quencher, a FRET biosensor, mass labels for mass-spectrometry, enzymes, antigens, metal particles, and dyes.
Item 7. The method according to any one of Items 1 to 6, wherein said PLPro is from HCoV-OC43, SARS-CoV-1, HCoV-HKU1, MERS-CoV or SARS-CoV-2.
Item 8. The method according to any one of Items 1 to 7, wherein said method is conducted in a high-throughput format.
Item 9. A method for identifying a modulator of the activity of a betacoronavirus papain-like protease PLpro, comprising the steps of contacting a peptide substrate for said PLpro with said PLpro in the presence of at least one candidate modulator, detecting cleaving of said peptide substrate by said PLpro, comparing cleaving of said peptide substrate in the absence of the at least one candidate modulator, and identifying a modulator of the activity of said PLpro based on said comparing, wherein said peptide substrate comprises at least one basic amino acid in positions P1 and/or P1' according to the Schechter and Berger nomenclature.
Item 10. The method according to Item 9, wherein said modulator is an inhibitor of the activity of a betacoronavirus papain-like protease PLpro.
Item 11. The method according to Item 9 or 10, wherein said peptide substrate comprises at least one additional basic amino acids in positions P2 to P3 and P2' to P4' thereof, and preferably one or two additional basic amino acids in positions P3' and/or P4'.
Item 12. The method according to any one of Items 9 to 11, wherein said basic amino acids are selected from lysine and/or arginine, and/or wherein said peptide substrate comprises a basic amino acids in both positions P1 and P1' thereof, preferably lysine/lysine or arginine/arginine.
Item 13. The method according to any one of Items 9 to 12, wherein said peptide substrate has a length of between 4 to 20 amino acids, preferably of between 4 and 10 amino acids, and wherein preferably P1 and P1' are located in the middle of said substrate.
Item 14. The method according to any one of Items 9 to 13, wherein said peptide substrate has an amino acid sequence of a naturally occurring protein, or wherein said peptide is synthetically produced, wherein preferably said peptide substrate comprises human SUMO1 or a fragment thereof comprising the amino acid sequence QRQGV (SEQ ID NO: 1).
Item 15. The method according to any one of Items 9 to 14, wherein said peptide substrate comprises a label suitable for detecting a cleaving thereof, such as, for example, a fluorophore and quencher, a FRET biosensor, mass labels for mass-spectrometry, enzymes, antigens, metal particles, and dyes.
Item 16. The method according to any one of Items 9 to 15, wherein said PLpro is from HCoV-OC43, SARS-CoV-1, HCoV-HKU1, MERS-CoV or SARS-CoV-2.
Item 17. The method according to any one of Items 9 to 16, wherein said method is conducted in a high-throughput format.
Item 18. The method according to any one of Items 9 to 17, wherein said modulator is at least one compound selected from a proteinaceous domain, a small molecule, a peptide, antibodies, an environmental substance, probiotic, toxin, aerosol, medicine, nutrient, galenic composition, plant extract, volatile compound, homeopathic substance, incense, pharmaceutical drug, vaccine, a compound or compound mixture derived from organisms, for example animals, plants, fungi, bacteria, archaea, a chemical compound, a compound used in food or cosmetic industry, and a library of said compounds, and preferably a small molecule.
Item 19. A method for identifying at least one compound as suitable for the treatment of a betacoronaviral infection, comprising performing the method according to any one of Items 9 to 18, and detecting a reduction of betacoronaviral infection in the presence of said modulator compared to infection in the absence of said modulator.
Item 20. The method according to Item 19, wherein said betacoronaviral infection is with HCoV-OC43, SARS-CoV-1, HCoV-HKU1, MERS-CoV or SARS-CoV-2.
Item 21. The method according to Item 12 or 13, wherein the peptide substrate is human SUMO1 or a fragment thereof comprising the amino acid sequence QRQGV (SEQ ID NO: 1).
Item 22. A method for producing a pharmaceutical composition effective against betacoronaviral infection, comprising performing a method according to Item 19 to 21, and suitably formulating said compound as identified into a pharmaceutical composition.
Item 23. A method for treating a betacoronaviral infection in a subject in need thereof, comprising administering a pharmaceutical composition as produced according to Item 22 to said subject.
Item 24. A kit comprising materials for performing a method according to any one of Items 1 to 21 in one or separate containers, optionally together with auxiliary agents and/or instructions for performing said method.
Item 25. Use of the kit according to Item 24 for a method according to any one of Items 1 to 19.
Item 26. An inhibitor of the activity of a betacoronavirus papain-like protease PLpro on SUMO1 for use in the treatment or prevention of a betacoronaviral infection in a subject.

The present invention will now be described further in the following examples with reference to the accompanying Figures, nevertheless, without being limited thereto. For the purposes of the present invention, all references as cited herein are incorporated by reference in their entireties.
Figure 1 shows the Schechter and Berger nomenclature for specificity of proteases. Figure taken from https://prosper.erc.monash.edu.au/methodology.html
Figure 2 shows the determination of the biochemical in vitro preferences of proteases using proteome-derived peptide libraries. Modified after (14).
Figure 3 shows the determination of preferences for recombinantly produced SARS CoV2 PL-Pro using proteome-derived peptide libraries at pH 8.0; Enzyme/Library Ratio: 1/1; 50 mM HEPES pH 8, 5 mM DTT, Incubation o/n 37°C. Arrows indicate cleavage site.

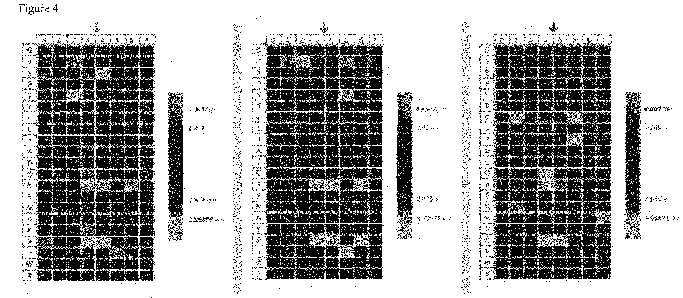

Figure 4 shows the determination of preferences for recombinantly produced SARS CoV2 PL-Pro using proteome-derived peptide libraries at pH 6.0; Enzyme/Library Ratio: 1/1; 100 mM phosphate buffer pH 6.0, 5 mM DTT, 150 mM NaCL,, 5 mM DTT, Incubation o/n 37°C. Arrows indicate cleavage site.

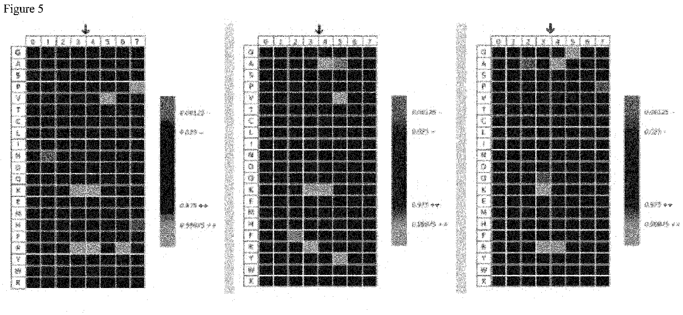

Figure 5 shows the determination of preferences for SARS CoV2 PL-Pro (Boston Biochem) using proteome-derived peptide libraries at pH 8.0; Enzyme/Library Ratio: 1/1; 50 mM HEPES pH 8, 5 mM DTT, Incubation o/n 37°C. Arrows indicate cleavage site.

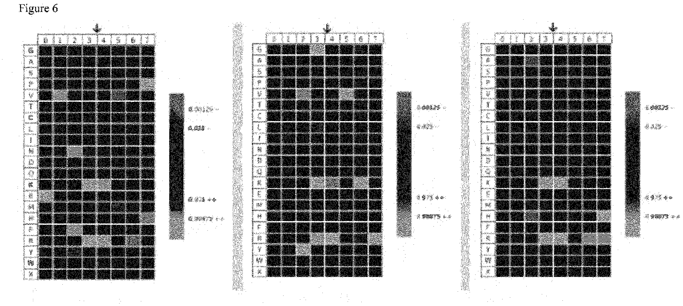

Figure 6 shows the determination of preferences for SARS CoV2 PL-Pro (Boston Biochem) using proteome-derived peptide libraries at pH 6.0; Enzyme/Library Ratio: 1/1; 100 mM phosphate buffer pH 6.0, 5 mM DTT, 150 mM NaCL, 5 mM DTT, Incubation o/n 37°C. Arrows indicate cleavage site.
Figure 7 shows that in the vicinity of the active center of SARS CoV2 PL-Pro (red amino acids, Cys111->Ser111; Asp286) there are numerous acidic amino acids capable of eventually interacting with basic amino acids. Structure 7cjd.pdb according to (15); yellow/green for a distinction of monomers; blue: aspartate and glutamate. Arrows indicate cleavage site.
Figure 8 shows a schematic overview of the processing of SUMO1 by SARS CoV2 PL-Pro.

### Examples

### Example 1

SARS CoV2 PL-Pro protease was produced as a recombinant enzyme in the laboratory of the inventors, or commercially obtained as an external control (Boston Biochem).

An unbiased specificity screening for the endoproteolytic activity of SARS CoV2 PL-Pro was performed. The approach as used is schematically depicted in Figure 2. Peptide libraries were produced with GluC or chymotrypsin in order to be able to also show an eventual preference for basic amino acids.

Using the recombinant protease as produced, at pH 6.0 and 8.0 a clear preference for basic amino acids (Arg or Lys) in P1 or P1' (Figure 3, Figure 4) was found; the same result was found using SARS CoV2 PL-Pro as commercially obtained (Figure 5, Figure 6).

A structural analysis of PLpro shows numerous acidic amino acids in the vicinity of the active center that can contribute to the binding of basic amino acids (Figure 7).

### Example 2

It was tested in an additional experiment, whether SARS CoV2 PL-Pro can also cleave proteins instead of peptides at basic residues in position P1 or P1'. A lung-cell pellet was lysed with the detergent RapiGest; an additional lysate was mechanically lysed only in 10 mM HEPES. Both lysates were digested with PLpro (50 mM HEPES pH 8; Enzyme/protein ratio: 1/10; incubation o/n 37°C) and subsequently GluC. Semi-specific peptides showing > 50 % enrichment upon SARS CoV2 PL-Pro incubation were taken into account.

Basic amino acids can be seen in P1 and P1' (Figure 8).

**Table 1 summarizes the basic amino acids as identified in substrates around the cleavage site between position 3 and 4 of the table.**

| **P3** | **P2** | **P1** | **P1'** | **P2'** | **P3'** | **P4'** |
|---|---|---|---|---|---|---|
| R | | K | K | R | K | R |
| | Q | R | R | | R | H |
| | A | | | V | | |
| V | V | G | A | G | | P |
| C | F | | S | A | | |
| | S | | | Y | | |
| | N | | | C | | |
| | Y | | Q | | V | |

### Example 3

The processing of SUMO1 by PLpro was analyzed, and Figure 8 shows the schematic summary of the results as obtained; processing of SUMO1 takes place at position 54/55, and/or at 56 and/or 57. According to the sequence of SUMO1 (P63165; NM_003352; Homo sapiens small ubiquitin like modifier 1 (SUMO1), transcript variant 1, mRNA), processing seems to take place either before or after (in the vicinity of) the arginine in the sequence QR₅₄QGV (SEQ ID NO: 1).

### References as cited

1. Anirudhan, V. et al. Targeting SARS-CoV-2 Viral Proteases as a Therapeutic Strategy to Treat COVID-19. J Med Virol (2021).
2. Shin, D. et al. Papain-like protease regulates SARS-CoV-2 viral spread and innate immunity. Nature 587, 657-662 (2020).
3. Schechter, I. & Berger, A. On the size of the active site in proteases. I. Papain. Biochem Biophys Res Commun 27, 157-162 (1967).
4. Rut, W. et al. Activity profiling and crystal structures of inhibitor-bound SARS-CoV-2 papain-like protease: A framework for anti-COVID-19 drug design. Sci Adv 6 (2020).
5. Alamri, M.A. et al. Discovery of human coronaviruses pan-papain-like protease inhibitors using computational approaches. JPharm Anal 10, 546-559 (2020).
6. Pitsillou, E. et al. Identification of Small Molecule Inhibitors of the Deubiquitinating Activity of the SARS-CoV-2 Papain-Like Protease: in silico Molecular Docking Studies and in vitro Enzymatic Activity Assay. Front Chem 8, 623971 (2020).
7. Penalver, L. et al. A Ligand Selection Strategy Identifies Chemical Probes Targeting the Proteases of SARS-CoV-2. Angew Chem Int Ed Engl (2020).
8. Mouffouk, C. et al. Flavonols as potential antiviral drugs targeting SARS-CoV-2 proteases (3CL(pro) and PL(pro)), spike protein, RNA-dependent RNA polymerase (RdRp) and angiotensin-converting enzyme II receptor (ACE2). Eur J Pharmacol 891, 173759 (2021).
9. Mitra, D. et al. Molecular docking and simulation studies of natural compounds of Vitex negundo L. against papain-like protease (PL(pro)) of SARS CoV-2 (coronavirus) to conquer the pandemic situation in the world. JBiomol Struct Dyn, 1-22 (2021).
10. Kouznetsova, V.L. et al. Potential COVID-19 papain-like protease PL(pro) inhibitors: repurposing FDA-approved drugs. PeerJ 8, e9965 (2020).
11. Diniz, L.R.L. et al. Bioactive Terpenes and Their Derivatives as Potential SARSCoV-2 Proteases Inhibitors from Molecular Modeling Studies. Biomolecules 11 (2021).
12. Siddiqui, S. et al. Virtual screening of phytoconstituents from miracle herb nigella sativa targeting nucleocapsid protein and papain-like protease of SARS-CoV-2 for COVID-19 treatment. J Biomol Struct Dyn, 1-21 (2020).
13. Welker, A. et al. Structure-Activity Relationships of Benzamides and Isoindolines Designed as SARS-CoV Protease Inhibitors Effective against SARS-CoV-2. ChemMedChem 16, 340-354 (2021).
14. Biniossek, M.L. et al. Identification of Protease Specificity by Combining Proteome-Derived Peptide Libraries and Quantitative Proteomics. Mol Cell Proteomics 15, 2515-2524 (2016).
15. Gao, X. et al. Crystal structure of SARS-CoV-2 papain-like protease. Acta Pharm Sin B 11, 237-245 (2021).

## Claims

1. A method for determining the activity of a betacoronavirus papain-like protease (PLpro), comprising the steps of contacting a peptide substrate for said PLpro with said PLpro, measuring cleaving of said peptide substrate by said PLpro, and determining the activity of said PLpro based on said cleaving, wherein said peptide substrate comprises at least one basic amino acid in positions PI and/or P1' according to the Schechter and Berger nomenclature.

2. A method for identifying a modulator of the activity of a betacoronavirus papain-like protease (PLpro), comprising the steps of contacting a peptide substrate for said PLpro with said PLpro in the presence of at least one candidate modulator, detecting cleaving of said peptide substrate by said PLpro, comparing cleaving of said peptide substrate in the absence of the at least one candidate modulator, and identifying a modulator of the activity of said PLpro based on said comparing, wherein said peptide substrate comprises at least one basic amino acid in positions PI and/or P1' according to the Schechter and Berger nomenclature.

3. The method according to claim 2, wherein said modulator is an inhibitor of the activity of a betacoronavirus papain-like protease PLpro.

4. The method according to any one of claims 1 to 3, wherein said peptide substrate comprises at least one additional basic amino acids in positions P2 to P3 and P2' to P4' thereof, and preferably one or two additional basic amino acids in positions P3' and/or P4'.

5. The method according to any one of claims 1 to 4, wherein said basic amino acids are selected from lysine and/or arginine, and/or wherein said peptide substrate comprises a basic amino acids in both positions P1 and P1' thereof, preferably lysine/lysine or arginine/arginine.

6. The method according to any one of claims 1 to 5, wherein said peptide substrate has a length of between 4 to 20 amino acids, preferably of between 4 and 10 amino acids, and wherein preferably P1 and P1' are located in the middle of said substrate.

7. The method according to any one of claims 1 to 6, wherein said peptide substrate has an amino acid sequence of a naturally occurring protein, or wherein said peptide is synthetically produced, wherein preferably said peptide substrate comprises human SUMO1 or a fragment thereof comprising the amino acid sequence QRQGV (SEQ ID NO: 1).

8. The method according to any one of claims 1 to 7, wherein said peptide substrate comprises a label suitable for detecting a cleaving thereof, such as, for example, a fluorophore and quencher, a FRET biosensor, mass labels for mass-spectrometry, enzymes, antigens, metal particles, and dyes.

9. The method according to any one of claims 1 to 8, wherein said PLpro is from HCoV-OC43, SARS-CoV-1, HCoV-HKU1, MERS-CoV or SARS-CoV-2.

10. The method according to any one of claims 1 to 9, wherein said method is conducted in a high-throughput format.

11. The method according to any one of claims 2 to 10, wherein said modulator is at least one compound selected from a proteinaceous domain, a small molecule, a peptide, antibodies, an environmental substance, probiotic, toxin, aerosol, medicine, nutrient, galenic composition, plant extract, volatile compound, homeopathic substance, incense, pharmaceutical drug, vaccine, a compound or compound mixture derived from organisms, for example animals, plants, fungi, bacteria, archaea, a chemical compound, a compound used in food or cosmetic industry, and a library of said compounds, and preferably a small molecule.

12. A method for identifying at least one compound as suitable for the treatment of a betacoronaviral infection, comprising performing the method according to any one of claims 2 to 11, and detecting a reduction of betacoronaviral infection in the presence of said modulator compared to infection in the absence of said modulator.

13. The method according to claim 12, wherein said betacoronaviral infection is with HCoV-OC43, SARS-CoV-1, HCoV-HKU1, MERS-CoV or SARS-CoV-2.

14. The method according to claim 12 or 13, wherein the peptide substrate is human SUMO1 or a fragment thereof comprising the amino acid sequence QRQGV (SEQ ID NO: 1).

15. A method for producing a pharmaceutical composition effective against betacoronaviral infection, comprising performing a method according to claim 12 to 14, and suitably formulating said compound as identified into a pharmaceutical composition.

16. A method for treating or preventing a betacoronaviral infection in a subject in need thereof, comprising administering a pharmaceutical composition as produced according to claim 15 to said subject.

17. A kit comprising materials for performing a method according to any one of claims 1 to 14 in one or separate containers, optionally together with auxiliary agents and/or instructions for performing said method.

18. Use of the kit according to claim 17 for a method according to any one of claims 1 to 14.

19. An inhibitor of the activity of a betacoronavirus papain-like protease PLpro on SUMO1 for use in the treatment or prevention of a betacoronaviral infection in a subj ect.
